(19) Europäisches Patentamt
European Patent Office
Office européen des brevets

(11)  **EP 2 097 166 B1**

(12)  **FASCICULE DE BREVET EUROPEEN**

(45) Date de publication et mention
de la délivrance du brevet:
**05.04.2017  Bulletin 2017/14**

(51) Int Cl.:
*B01J 21/04* (2006.01)  *B01J 23/08* (2006.01)
*B01J 23/14* (2006.01)  *B01J 23/40* (2006.01)
*C07C 5/32* (2006.01)  *C07C 5/333* (2006.01)
*C07C 11/02* (2006.01)

(21) Numéro de dépôt: **07871817.8**

(22) Date de dépôt: **07.12.2007**

(86) Numéro de dépôt international:
**PCT/FR2007/002022**

(87) Numéro de publication internationale:
**WO 2008/084147 (17.07.2008 Gazette 2008/29)**

(54) **PROCEDE DE DESHYDROGENATION EN PRESENCE D'UN CATALYSEUR BIMETALLIQUE OU MULTI-METALLIQUE AYANT UN INDICE DE BIMETALLICITE ET UNE CAPACITE D'ADSORPTION D'HYDROGENE OPTIMISES**

DEHYDRIERFERFAHREN IN GEGENWART EINES BIMETALL- ODER MULTIMETALLKATALYSATORS MIT OPTIMIERTEM BIMETALLINDEX UND WASSERSTOFFABSORPTIONSFÄHIGKEIT

METHOD FOR DEHYDROGENATION IN THE PRESENCE OF A BI-METALLIC OR MULTI-METALLIC CATALYST HAVING OPTIMISED BI-METALLIC INDEX AND HYDROGEN ADSORPTION CAPACITY

(84) Etats contractants désignés:
**AT BE BG CH CY CZ DE DK EE ES FI FR GB GR HU IE IS IT LI LT LU LV MC MT NL PL PT RO SE SI SK TR**

(30) Priorité:  **22.12.2006  FR 0611412**

(43) Date de publication de la demande:
**09.09.2009  Bulletin 2009/37**

(73) Titulaire: **IFP Énergies nouvelles**
**92852 Rueil-Malmaison Cedex (FR)**

(72) Inventeurs:
• **LE PELTIER, Fabienne**
**F-92500 Rueil Malmaison (FR)**
• **LACOMBE, Sylvie**
**F-69390 Vernaison (FR)**
• **CHAU, Christophe**
**F-69360 Communay (FR)**
• **MORIN, Stéphane**
**F-69230 Saint Genis Laval (FR)**
• **FISCHER, Lars**
**F-38200 Vienne (FR)**
• **REVEL, Renaud**
**F-38200 Serpaize (FR)**

(56) Documents cités:
**EP-A- 0 568 303      EP-A- 1 182 180**
**EP-A1- 0 248 130     US-A- 3 531 543**
**US-A- 4 786 625**

Remarques:
Le dossier contient des informations techniques présentées postérieurement au dépôt de la demande et ne figurant pas dans le présent fascicule.

EP 2 097 166 B1

**Description**

**DOMAINE DE L'INVENTION**

La présente invention concerne le domaine de la deshydrogenation de charges hydrocarbonées en présence d'un catalyseur bimétallique ou multi-métallique à base d'un métal noble.

La déshydrogénation des hydrocarbures est un procédé permettant de synthétiser de nombreux produits chimiques servant de base à la préparation de produits manufacturés tels que des essences à haut indice d'octane, des plastiques ou des plastifiants, des détergents, des caoutchoucs synthétiques, et différents additifs pour plastiques, lubrifiants ou adhésifs.

**ART ANTERIEUR**

L'art antérieur décrit de nombreux catalyseurs à base de métaux nobles du groupe VIII, d'alcalins et de promoteurs. Néanmoins ces formulations peuvent encore faire l'objet de perfectionnement permettant d'améliorer leurs performances, notamment dans le but de minimiser encore plus les réactions secondaires telles que l'isomérisation ou le craquage, ou d'obtenir des catalyseurs plus stables dans le temps, ou encore de favoriser la conversion de molécules difficiles à déshydrogéner.

Le brevet US 4,786,625 décrit des catalyseurs de déshydrogénation comprenant un métal du groupe du platine et un élément modificateur sélectionné dans le groupe composé par l'étain, le germanium, le rhénium ou leurs mélanges déposés sur un oxyde réfractaire. présentant un diamètre d'un moins 850 microns dans lequel le platine est déposé en surface du support et le modificateur imprégné uniformément dans le support.

La demande de brevet US 2005/0033101 A1 décrit un procédé de déshydrogénation dans lequel la charge est mise en contact avec un catalyseur comprenant au moins un métal noble du groupe VIII, un alcalin ou un alcalino-terreux et un composé sélectionné parmi l'étain, le germanium, le plomb, l'indium, le gallium, le thallium ou leur mélange. Ces différents composants du catalyseurs sont déposés sur un support à base d'alumine comprenant essentiellement de l'alumine thêta.

Le brevets US 6,600,082 B2 décrit un procédé de déshydrogénation de composés organiques mettant en oeuvre un catalyseur à base de métaux du groupe VIII et d'étain, dans lequel une partie de l'étain est sous forme réduite à l'état métallique et le catalyseur est dans un état partiellement oxydé obtenu d'une manière contrôlée.

**RESUME DE L'INVENTION**

L'invention concerne un procédé de déshydrogénation d'une charge hydrocarbonée en présence d'un catalyseur comprenant du platine et un promoteur X1 constitué par l'étain, et éventuellement un promoteur X2 constitué par l'indium, un composé alcalin ou alcalino-terreux et un support poreux, dans lequel le rapport atomique X1/M et éventuellement le rapport X2/M est compris entre 0,3 et 8, le rapport $H_{ir}/M$ mesuré par adsorption d'hydrogène est supérieur à 0,40 et l'indice IBM de bimétallicité mesuré par titrage hydrogène/oxygène est supérieur à 108.

**DESCRIPTION DETAILLEE DE L'INVENTION**

La présente invention concerne un procédé de déshydrogénation d'une charge hydrocarbonée de paraffine à une pression comprise entre 0,02 et 2 MPa, à une température comprise entre 400 et 800°C, à un débit massique de charge traitée par unité de masse de catalyseur compris entre 0,5 et 200 kg/(kgcat.h) et un rapport molaire hydrogène/hydrocarbure compris entre 0 et 20, en présence d'un catalyseur comprenant un métal noble M de platine dans une teneur de 0,01 à 10% pds ; au moins un promoteur X1 constitué par l'étain dans une teneur de 0,005 à 10% pds ; un composé alcalin ou alcalino-terreux ; et un support poreux, dans lequel le rapport X1/M est compris entre 0,3 et 8, le rapport $H_{ir}/M$ mesuré par chimisorption d'hydrogène de façon irréversible telle que décrite dans la description est supérieur à 0,40 et l'indice IBM de bimétallicité mesuré par titrage hydrogène/oxygène tel que décrit dans la description est supérieur à 108, et dans lequel le procédé de préparation du catalyseur comprend les étapes suivantes:

a) introduction du promoteur X1 dans le support ou sur le support dans une teneur de 30 à 70% pds de la quantité totale de X1,

b) étape optionnelle de séchage du produit obtenu à l'issue de l'étape a,

c) calcination du produit obtenu à l'étape a ou éventuellement à l'étape b à une température de préférence comprise entre 350 et 650°C,

d) dépôt du platine,

e) séchage optionnel sous atmosphère neutre ou contenant de l'oxygène, à une température modérée et n'excédant pas de préférence 150°C,

f) dépôt d'une fraction complémentaire du promoteur X1, dans une teneur de 30 à 70% pds de la quantité totale de X1, sur le produit obtenu à l'étape d ou e, ladite fraction est déposée par mise en contact avec une solution de précurseur de tétrachlorure d'étain,

g) séchage optionnel du produit obtenu à l'étape f,

h) calcination du produit obtenu à l'étape f ou g, cette calcination est de préférence menée en présence d'air et cet air peut également être enrichi en oxygène ou en azote ;

m) deshalogénation en phase gazeuse en présence d'une fraction d'oxygène dépassant les 2% volume, à une température comprise entre 300 et 600°C de façon à obtenir une teneur finale en halogène comprise entre 300 et 1 000 ppm poids ;

n) introduction du composé alcalin ou alcalino-terreux ;

o) séchage optionnel sous une atmosphère neutre ou contenant de l'oxygène, à une température modérée, du produit obtenu à l'étape n ;

p) calcination du produit obtenu à l'étape n ou o, cette calcination est menée en présence d'air et cet air pouvant également être enrichi en oxygène ou en azote.

Dans le catalyseur selon l'invention le rapport atomique X1/M est de préférence compris entre 0,6 et 6, de manière plus préférée compris entre 0,7 et 5, de manière plus préférée entre 0,8 et 4, de manière encore très préférée entre 1,2 et 2,9, voire entre 1,4 et 2,5.

Le catalyseur selon l'invention peut en outre éventuellement comprendre au moins un promoteur X2 constitué par l'indium. De préférence le rapport X2/M est alors compris entre 0,3 et 8, de manière plus préférée entre 0,6 et 6, de manière encore plus préférée entre 0,7 et 5, de manière beaucoup plus préférée entre 0,8 et 4, de manière très préférée entre 1,0 et 2,9, voire entre 1,2 et 2,6.

Le catalyseur selon l'invention contient préférentiellement de 0,01 à 10 % poids, de manière plus préférée de 0,02 à 2 % poids, et de manière très préférée de 0,05 à 0,5 % poids de platine.

La teneur en promoteur X1 ou X2, est de préférence comprise entre 0,005 à 10 %poids et de manière plus préférée entre 0,01 et 5% poids et de manière très préférée entre 0,1 et 2% poids.

Selon une autre variante, le catalyseur selon l'invention peut donc comprendre uniquement un promoteur X1 constitué par l'étain.

Selon une autre variante, le catalyseur selon l'invention peut comprendre à la fois un promoteur X1 constitué par l'étain et un promoteur X2 constitué par l'indium.

Le composé alcalin est de préférence sélectionné dans le groupe constitué par: le lithium, le sodium, le potassium, le rubidium et le césium. Le lithium, le sodium ou le potassium sont les alcalins très préférés et le lithium ou le potassium sont les alcalins encore plus préférés.

La teneur en composé alcalin est de préférence comprise entre 0,05 et 10 % poids, de manière plus préférée comprise entre 0,1 et 5 % poids, et de manière encore plus préférée comprise entre 0,15 et 2 % poids.

Le composé alcalino-terreux est de préférence sélectionné dans le groupe constitué par: le magnésium, le calcium, le strontium ou le baryum. Le magnésium ou le calcium sont les alcalino-terreux très préférés et le magnésium est l'alcalino-terreux le plus préféré. La teneur en composé alcalino-terreux est de préférence comprise entre 0,05 et 10 %poids, de manière plus préférée comprise entre 0,1 et 5 % poids, et de manière encore plus préférée comprise entre 0,15 et 2 % poids.

Le catalyseur selon l'invention présente par ailleurs une capacité d'adsorption d'hydrogène telle que le rapport entre la quantité d'hydrogène irréversiblement adsorbée et le métal du groupe du platine (également appelé rapport atomique $H_{irr}/M$) est supérieur à 0,4, de préférence supérieur à 0,43, de manière plus préféré comprise entre 0,43 et 0,9, de manière encore plus préférée comprise entre 0,45 et 0,65, et de manière très préférée comprise entre 0,45 et 0,6.

Mesure du rapport $H_{irr}/M$

Le rapport $H_{ir}/M$ d'un catalyseur selon l'invention peut être déterminé au moyen de la technique de chimisorption d'hydrogène. Cette technique est connue de l'Homme du métier et décrite par exemple au chapitre 5 page 127 et suivantes du livre de revue intitulé: "Catalytic Naphta Reforming, Science and Technology, de G.J. Antos, A.M. Aitani et J.M. Parera, Éditeur Marcel Decker, 1995. Elle permet en effet de caractériser des systèmes complexes à base de platine où d'autres métaux et d'un ou plusieurs promoteurs.

Différents protocoles ont été proposés dans la littérature pour déterminer les quantités d'hydrogène chimisorbé. L'hydrogène peut être chimisorbé par un catalyseur métallique de façon réversible ou irréversible. Le protocole détaillé ci-après est préféré pour la détermination du rapport $H_{ir}/M$ selon l'invention qui fait intervenir la quantité $H_{ir}$ d'hydrogène chimisorbé de façon irréversible.

Protocole de traitement de l'échantillon:

a) Calcination sous courant d'air sec 2 h à 500°C.

b) Passage à l'air à température ambiante pour chargement dans la cellule de mesure par volumétrie en moins de 10 minutes.

c) Réduction dans la cellule avec augmentation de la température de 20°C à 450°C en 1 h, maintien à 450°C durant 4 h sous courant d'hydrogène (50 mlitres.min$^{-1}$), retour à 25°C sous hydrogène, puis scellage de l'évent de la cellule.

Mesures d'adsorption:

L'appareillage utilisé est un appareillage de volumétrie en statique.

a) Désorption sous vide dynamique (10$^{-5}$ mbar soit 1 mPa) à 350°C, 3 h puis retour à 25°C sous vide.

b) Mesure de la quantité d'hydrogène adsorbé à 25°C sous une pression donnée d'hydrogène, après adsorption pendant 60 minutes.

Les phases a) et b) sont reproduites de manière à tracer l'isotherme d'adsorption entre environ 40 et 300 mbar (4 à 30kPa) Deux mesures sont réalisées en désorbant sous vide à 25°C durant 3 h qui permettent de mesurer la quantité d'hydrogène adsorbée de manière réversible.

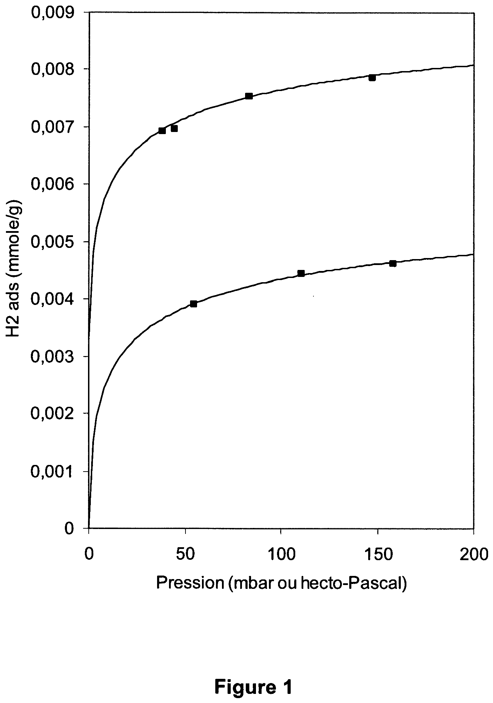

La figure 1 présente un exemple d'isotherme obtenue pour un catalyseur selon l'invention. La courbe du bas correspond à l'isotherme d'adsorption réversible et la courbe du haut représente l'isotherme d'adsorption totale.

Il est possible de modéliser l'isotherme d'adsorption, en admettant une adsorption dissociative de l'hydrogène sur le platine, selon l'équation de Langmuir, avec une partie irréversible $H_{ir}$ à 25°C et une partie réversible $H_{rev}$.

La quantité totale d'hydrogène adsorbée est définie par l'équation (I) ci-dessous:

$$Q_{ads} = H_{ir} + H_{rev}.(K_1.P)^{0.5}/(1+(K_1.P)^{0.5}) \qquad (I)$$

où P est la pression exprimée en mbar (ou hectopascal) et $K_1$ est la constante de Langmuir. La modélisation de l'isotherme d'adsorption permet donc de déterminer le paramètre $H_{ir}$

Indice de bimétallicité :

Le catalyseur optimisé selon l'invention présente un indice de bimétallicité (IBM) supérieur à 108. Cet indice défini ci-après est mesuré par la technique de titrage hydrogène/oxygène détaillée ci-après. De manière préférée cet indice IBM est supérieur à 110 et de manière très préférée supérieur à 115 voire à 120. Par ailleurs, selon une variante très préférée, cet indice est inférieur à 170, et de manière encore plus préférée inférieur à 160. Il est par ailleurs possible selon une autre variante préférée que cet indice soit compris entre 108 et 160, ou de façon très préférée entre 110 et 160, voire entre 110 et 150 ou encore entre 115 et 145.

L'indice de bimétallicité (IBM) est déterminé en exploitant d'une manière particulière la technique de titrage hydrogène-oxygène. Cette technique est connue de l'homme du métier pour déterminer la dispersion d'un métal, c'est-à-dire le rapport du nombre d'atomes de surface d'un métal au nombre total d'atomes de ce même métal. Elle est décrite par exemple au chapitre 5 pages 130 et 131 du livre de revue intitulé: "Catalytic Naphta Reforming, Science and Technology », de G.J. Antos, A.M. Aitani et J.M. Parera, Éditeur Marcel Decker, 1995. Elle consiste à chimisorber de l'oxygène sur un métal M contenu dans un catalyseur préalablement réduit et contenant donc une couche d'hydrogène chimisorbé. La réaction stoechiométrique représentant l'étape de titrage de l'hydrogène chimisorbé par l'oxygène est considérée comme étant la suivante:

M-H + 3/4 O$_2$ → M-O + ½ H$_2$O

La quantité d'oxygène consommée lors du titrage permet de déterminer la quantité de sites métalliques accessibles. Dans le cas d'un système bimétallique Pt-Sn supporté il a été montré, par exemple par Sharma et al dans Applied Catalysis A 168 (1998) 251, qu'en opérant deux cycles successifs de titrage d'oxygène, la quantité d'oxygène adsorbée lors du premier cycle était plus importante que celle adsorbée lors du deuxième cycle. Ceci est expliqué par le fait que lors du premier cycle, la réduction de l'étain contribue à la consommation d'oxygène, alors que le deuxième cycle reflète essentiellement la chimisorption d'oxygène sur les sites de platine accessibles.

Ce deuxième cycle peut être utilisé pour déterminer la dispersion du métal M. Dans le catalyseur selon l'invention, la dispersion du métal M est de préférence supérieure à 80%, de manière plus préférée supérieure à 90% et de manière très préférée supérieure à 95%.

L'indice de bimétallicité dit « IBM » utilisé comme critère dans la présente invention est calculé à partir du volume d'oxygène consommé lors du premier titrage hydrogène-oxygène. Il est calculé comme suit:

$$IBM = (V1 / 24041) * 4/3 / (0,01\ T_M / MM)$$

avec

> V1: volume d'oxygène consommé lors du premier titrage à 20°C sur le catalyseur préalablement réduit, exprimé en $cm^3$ par gramme de catalyseur.
> $T_M$: teneur en métal du groupe du platine dans le catalyseur, exprimée en %poids
> MM: masse molaire du du platine

L'indice de bimétallicité traduit le degré d'interaction du métal du groupe du platine avec tout promoteur présent sur le catalyseur. Le protocole expérimental de titrage hydrogène-oxygène préféré pour la détermination de l'indice de bimétallicité est le suivant:

> a) calcination de 2 grammes de catalyseur sous courant d'air sec (20ml/minute) avec un débit de 1 Nl/(g.h) pendant 2h à 500°C,
> b) retour à 20°C sous air sec,
> c) purge par un gaz inerte (20ml/minute), de préférence de l'hélium de pureté supérieure à 99,999%,
> d) passage sous hydrogène (20ml/minute), de préférence de pureté supérieure à 99,999% et réduction à 500°C pendant 2h
> e) retour à 20°C
> f) purge par un gaz inerte (20ml/minute), de préférence de l'hélium de pureté supérieure à 99,999%,
> g) pulses d'oxygène de volume 0,27 $cm^3$, de préférence de pureté supérieure à 99,995% et purifié au moyen d'un ou plusieurs tamis moléculaires (tamis 3A, 4A ou 13X) à 20°C jusqu'à ne plus avoir de consommation d'oxygène, soit après au moins 10 pulses de surface constante.

Le volume d'oxygène consommé lors de l'étape g) est utilisé pour le calcul de l'indice de bimétallicité.

Support du catalyseur selon l'invention:

Le support poreux employé dans le catalyseur selon l'invention est généralement un oxyde réfractaire sélectionné parmi les oxydes de magnésium, de titane, de zirconium, d'alumine, de silicium ou leur mélange. De préférence il s'agit de silice, d'alumine ou de silice-alumine, et de manière très préférée d'alumine.

Dans le cas des catalyseurs selon l'invention utilisés dans un procédé de déshydrogénation, le support poreux peut en sus être sélectionné parmi les zéolithes et tamis moléculaires ou les mélanges de zéolithes ou tamis moléculaires avec un des oxydes réfractaires cités ci-avant, et de préférence sélectionné parmi les supports de catalyseurs suivants: zéolithe X , zéolithe Y, modénite, faujasite, ZSM-5, ZSM-4, ZSM-8, EUO, mazzite.

Selon l'invention, ledit support poreux se présente avantageusement sous forme de billes, d'extrudés, de pastilles, de poudre ou d'agglomérats irréguliers et non sphériques dont la forme spécifique peut résulter d'une étape de concassage. De manière très avantageuse, ledit support se présente sous forme de billes ou d'extrudés ou d'agglomérats. Le volume poreux du support est de préférence compris entre 0,1 et 1,5 $cm^3/g$, de manière plus préférée compris entre 0,4 et 1,2 $cm^3/g$. Par ailleurs, ledit support poreux présente une surface spécifique comprise avantageusement entre 50 et 600 $m^2/g$, de préférence entre 70 et 250 $m^2/g$, voire entre 80 et 200 $m^2/g$.

Préparation du catalyseur:

Il a été découvert par la demanderesse que les catalyseurs selon l'invention étaient obtenus en particulier en déposant le promoteur X1 en deux fois (deux étapes distinctes), une première étape située avant l'introduction du platine, et une deuxième étape située après l'introduction du platine.

Le procédé de préparation du catalyseur selon l'invention comprend généralement les étapes suivantes :

> a) introduction du promoteur X1 dans le support ou sur le support dans une teneur de 30 à 70% pds de la quantité totale de X1,
> b) étape optionnelle de séchage du produit obtenu à l'issue de l'étape a,

c) calcination du produit obtenu à l'étape a ou éventuellement à l'étape b à une température de préférence comprise entre 350 et 650°C,

d) dépôt du platine,

e) séchage optionnel sous atmosphère neutre ou contenant de l'oxygène, à une température modérée et n'excédant pas de préférence 150°C,

f) dépôt d'une fraction complémentaire du promoteur X1, dans une teneur de 30 à 70% pds de la quantité totale de X1, sur le produit obtenu à l'étape d ou e, ladite fraction est déposée par mise en contact avec une solution de précurseur de tétrachlorure d'étain,

g) séchage optionnel du produit obtenu à l'étape f,

h) calcination du produit obtenu à l'étape f ou g, cette calcination est de préférence menée en présence d'air et cet air peut également être enrichi en oxygène ou en azote ;

m) deshalogénation en phase gazeuse en présence d'une fraction d'oxygène dépassant les 2% volume, à une température comprise entre 300 et 600°C de façon à obtenir une teneur finale en halogène comprise entre 300 et 1 000 ppm poids ;

n) introduction du composé alcalin ou alcalino-terreux ;

o) séchage optionnel sous une atmosphère neutre ou contenant de l'oxygène, à une température modérée, du produit obtenu à l'étape n ;

p) calcination du produit obtenu à l'étape n ou o, cette calcination est menée en présence d'air et cet air pouvant également être enrichi en oxygène ou en azote.

Plus précisément, pour la première étape (étape a) d'introduction du promoteur X1 (utilisé seul ou en mélange), X1 peut être incorporé dans le support, par exemple lors de la synthèse du support ou lors de la mise en forme du support. X1 peut également être introduit sur le support, par exemple par imprégnation du support préalablement mis en forme. X1 peut également être introduit pour partie lors de la synthèse ou mise en forme du support et pour partie par dépôt sur le support mis en forme. 30% à 70% en poids, de façon préférée 40% à 65% en poids et de façon encore plus préférée 50% à 65% en poids, de la quantité totale de X1 entrant dans la composition du catalyseur selon l'invention se trouvent ainsi introduits dans le support ou déposés sur le support lors de cette première étape d'introduction.

La fraction de X1 introduite lors de la synthèse du support est incorporée par toute technique connue de l'homme du métier. Sans être exhaustives, les techniques d'addition avant ou pendant la dissolution des précurseurs oxydes du support, avec ou sans mûrissement, peuvent convenir. L'introduction peut donc être simultanée ou successive au mélange des précurseurs du support.

Selon une variante de la méthode de préparation selon l'invention, le promoteur X1 est introduit lors de la synthèse du support selon une technique de type sol-gel. Selon une autre variante, le promoteur X1 est ajouté à un sol d'alumine.

Selon une troisième variante de mise en oeuvre de l'invention le promoteur X1 est introduit lors de la mise en forme du support selon les techniques de l'art antérieur de mise en forme du support telles que les procédures de mise en forme par extrusion ou par coagulation en gouttes (Oil-Drop selon la terminologie anglo-saxonne).

Dans le cas où la fraction de X1 est déposée sur le support, après sa mise en forme, elle peut être imprégnée au moyen de toute technique connue de l'homme de l'art, et de façon préférée par imprégnation d'une solution contenant un ou plusieurs précurseurs de X1. L'imprégnation peut être réalisée par excès de solution ou bien à sec (le volume de solution contenant X1 correspondant au volume poreux du support). L'imprégnation peut être réalisée en présence d'espèces agissant sur l'interaction entre le précurseur du promoteur X1 et le support. Ces espèces peuvent être par exemple, et sans être limitatif, des acides minéraux (HCl, HNO3) ou organiques (types acides carboxyliques ou polycarboxyliques), et des composés organiques de type complexants, comme cela est décrit par exemple dans les brevets US 6,872,300 B1 et US 6,291,394 B1. De manière préférée l'imprégnation est réalisée selon toute technique connue de l'homme de l'art permettant d'obtenir une répartition homogène du promoteur X1 au sein du catalyseur.

Les précurseurs du promoteur X1 peuvent être minéraux ou de type organométallique, éventuellement de type organométallique hydrosoluble. Les différents précurseurs du promoteur X1 constitué par l'étain peuvent être utilisés, seuls ou en mélange. En particulier, l'étain peut être choisi et ce, de façon non limitative, dans le groupe formé par les composés halogénés, hydroxydes, carbonates, carboxylates, sulfates, tartrates et nitrates. Ces formes de l'étain peuvent être introduites dans le milieu de préparation du catalyseur telles quelles ou générées in situ (par exemple par introduction d'étain et d'acide carboxylique). Les précurseurs de type organométallique à base d'étain peuvent être par exemple $SnR4$, où R représente un groupe alkyl, par exemple le groupement butyle, $Me_3SnCl$, $Me_2SnCl_2$, $Et_3SnCl$, $Et_2SnCl_2$, $EtSnCl_3$, $iPrSnCl_2$ et les hydroxydes $Me_3SnOH$, $Me_2Sn(OH)_2$, $Et_3SnOH$, $Et_2Sn(OH)_2$, les oxydes $(Bu_3Sn)_2O$, l'acétate $Bu_3SnOC(O)Me$. De façon préférée, les espèces halogénés, en particulier chlorées, d'étain seront utilisées. En particulier, $SnCl_2$ ou $SnCl_4$ seront employés avantageusement.

Quelle que soit la variante utilisée pour l'introduction d'une fraction du promoteur X1 lors de l'étape a, il peut être préféré d'effectuer un séchage du support (étape b) à la fin après ajout du promoteur. Ce séchage peut être effectué selon toute technique connue de l'homme du métier, par exemple à une température comprise entre 40°C et 200°C, de préférence entre 80°C et 180°C. Ce séchage peut être effectué avec une programmation de température et peut éventuellement comprendre des paliers de températures.

Le promoteur X1 constitué par l'étain ayant été introduit dans le support ou sur le support préalablement formé, le protocole de préparation des catalyseurs selon l'invention nécessite généralement une calcination avant le dépôt du platine (étape c). Cette calcination est de préférence menée à une température comprise entre 350 et 650°C et de préférence comprise entre 400 et 600°C et de manière encore plus préférée comprise entre 400 et 550°C. La montée en température peut être régulière ou inclure des paliers de températures intermédiaires, ces paliers étant atteints avec des vitesses de montée en température fixes ou variables. Ces montées en températures peuvent donc être identiques ou différer par leur vitesse (en degré par minute ou par heure). L'atmosphère de gaz utilisé durant la calcination contient de l'oxygène, de préférence entre 2 et 50% et de manière plus préférée entre 5 et 25%. De l'air peut donc également être utilisé lors de cette étape de calcination.

Après obtention du support contenant une fraction de X1 par rapport à la composition finale du catalyseur, un dépôt du platine est réalisé (étape d). Dans cette étape, le platine peut être introduit par imprégnation à sec ou en excès de solution, en utilisant un précurseur ou un mélange de précurseurs contenant du platine. L'imprégnation peut être réalisée en présence d'espèces agissant sur l'interaction entre le précurseur du platine et le support. Ces espèces peuvent être, et sans être limitatif, des acides minéraux (HCl, HNO3) ou organiques (types acides carboxyliques ou polycarboxyliques), et des composés organiques de type complexants. De manière préférée l'imprégnation est réalisée selon toute technique connue de l'homme de l'art permettant d'obtenir une répartition homogène du platine au sein du catalyseur.

Les précurseurs du platine font partie du groupe suivant, sans que cette liste ne soit limitative: acide hexachloroplatinique, acide bromoplatinique, chloroplatinate d'ammonium, chlorures de platine, dichlorocarbonyl dichlorure de platine, chlorure de platine tétra-amine.

A ce stade, le support contenant X1 (une fraction de la quantité totale en X1 visée dans le catalyseur final) et le platine est éventuellement séché (étape e), sous atmosphère neutre ou contenant de l'oxygène (de l'air pouvant être utilisé), à une température modérée et n'excédant pas de préférence 150°C. De préférence, le séchage est mis en oeuvre à une température inférieure à 100°C et sur une durée de quelques minutes à quelques heures.

A l'issue de cette étape éventuelle de séchage, une fraction complémentaire du promoteur X1 constitué par l'étain est déposée sur le produit obtenu à l'étape d ou e (étape f). Plus précisément, pour l'introduction de l'espèce X1 concernant cette étape, 30% au moins et 70% au plus, et de façon préférée 35% au moins et 60% au plus, voire 35% au moins et 50% au plus, de la quantité totale de X1 entrant dans la composition du catalyseur selon l'invention se trouvent ainsi introduits lors de cette deuxième étape d'introduction de X1. La fraction complémentaire du promoteur X1 est déposée par mise en contact d'une solution contenant le précurseur.

Dans cette étape, le tétrachlorure d'étain $SnCl_4$ sera exclusivement mis en oeuvre.

Un séchage optionnel du produit obtenu à l'étape f peut alors intervenir (étape g), sous atmosphère neutre ou contenant de l'oxygène (de l'air pouvant être utilisé), à une température modérée.

Cette étape est alors généralement suivie d'une calcination du produit obtenu à l'étape f ou g (étape h). Cette calcination est de préférence menée en présence d'air. Cet air peut également être enrichi en oxygène ou en azote. De préférence, la teneur en oxygène de ce gaz atteint 0,5 à 30% et de façon encore plus préférée 2 à 25%.

Cette calcination est menée à une température intervenant entre 350 et 600°C et de préférence entre 400 et 550°C et de manière encore plus préférée entre 450 et 550°C. La rampe de température doit être régulière et suffisamment rapide. Elle peut éventuellement contenir des paliers en température à partir de 350°C. La vitesse de montée en température est de préférence supérieure ou égale à 5°C/mn. Cette vitesse de montée peut être supérieure à 10°C/mn. De façon préférée, cette vitesse de montée en température ne sera pas inférieure à 2°C/mn.

Le promoteur X2 optionnel peut être introduit à une ou plusieurs étapes de la préparation du catalyseur. Il peut par exemple être introduit avant, pendant ou après la première étape d'introduction de X1 (étape a, lors de la mise en forme du support ou par dépôt sur le support), seul ou en mélange. X2 peut également être introduit entre l'étape de calcination du support (étape c) et l'étape d'introduction du métal du groupe du platine (étape d). Une autre possibilité d'introduction de X2 est de l'incorporer avant l'addition de la seconde fraction de X1 (étape f), il peut enfin être introduit avant l'étape de calcination finale (étape h). Le promoteur X2 peut être introduit en une ou plusieurs fois, à condition de respecter les conditions d'introduction citées précédemment. Le promoteur X2 peut également être introduit par imprégnation lors d'une étape additionnelle (étape i) située après l'étape h, l'étape i est alors généralement suivie d'étapes j et k respectivement de séchage et de calcination du catalyseur dans les conditions détaillées précédemment pour les étapes de séchage e ou g ou de calcination h.

Dans le cas où un promoteur X2 est présent, le procédé de préparation selon l'invention peut donc comprendre en outre et avantageusement une étape additionnelle d'introduction d'un promoteur X2 située avant l'étape a, ou entre l'étape c et l'étape d ou juste avant l'étape f ou l'étape h, ou encore après l'étape h.

Le promoteur X2 optionnel sélectionné dans le groupe constitué par l'indium peut être introduit au moyen de toute technique connue de l'homme du métier. Les échanges ioniques, imprégnations à sec ou en excès de solution sont appropriés lorsqu'il s'agit de dépôt sur le support (modifié par X1 ou non). Dans le cas d'une incorporation lors de la préparation du support, le promoteur X2 peut être ajouté par mélange, coprécipitation, dissolution sans être limitatif. Des précurseurs, seuls ou en mélange, de nitrate, chlorures ou bromures d'indium conviennent. D'autres précurseurs peuvent être également utilisés.

Sur un échantillon du catalyseur contenant le platine et le promoteur X1 ou les promoteurs X1 et X2 , l'indice de bimétallicité et le rapport $H_{ir}$/Pt chimisorption d'hydrogène sont déterminés après la calcination "finale" décrite ci-dessus, et avant une éventuelle deshalogénation et avant le dépôt d'un métal alcalin ou alcalino-terreux qui sont décrits dans ce qui suit.

La partie du catalyseur non utilisée pour ces caractérisations est soumise à une deshalogenation (étape m) et à un dépôt d'un métal alcalin ou alcalino-terreux (étape n).

Si le dépôt du platine et du métal X a été effectué en introduisant des composés halogénés, on peut procéder à une deshalogénation (étape m) dont les conditions opératoires peuvent être choisies parmi toutes les méthodes connues de l'homme du métier. On peut extraire l'halogène en phase liquide ou en phase gazeuse. De façon préférée, l'extraction de l'halogène se fait en phase gazeuse en présence d'une fraction d'oxygène dépassant les 2 % volume, à des températures comprises entre 300°C et 600°C, voire 400 à 550°C. De manière encore plus préférée, la phase gazeuse est saturée avec une phase aqueuse qui peut contenir un composé basique, tel que l'ammoniaque ou des amines. La durée du traitement est généralement de quelques heures, elle est ajustée de façon à obtenir une teneur finale en halogène comprise entre 100 ppm poids et 5000 ppm poids, de façon préférée entre 200 et 2000 ppm poids, voire entre 300 et 1000 ppm poids.

L'alcalin ou alcalino-terreux est par la suite être introduit (étape n) au moyen de toute technique connue de l'homme du métier. Les échanges ioniques, imprégnations à sec ou en excès de solution sont appropriées. Différents précurseurs peuvent être choisis, seuls ou en mélange et ce, de façon non limitative, dans le groupe formé par les composés hydroxydes, carbonates, carboxylates, sulfates, tartrates et nitrates. De manière préférée l'imprégnation est réalisée selon toute technique connue de l'homme du métier permettant d'obtenir une répartition homogène de l'alcalin ou alcalino-terreux au sein du catalyseur.

Un séchage optionnel peut par la suite intervenir, sous atmosphère neutre ou contenant de l'oxygène (de l'air pouvant être utilisé), à une température modérée. Cette étape est alors suivie d'une dernière calcination. Cette calcination est menée en présence d'air. Cet air peut également être enrichi en oxygène ou en azote. De préférence, la teneur en oxygène de ce gaz atteint 0.5 à 30% et de façon encore plus préférée 2 à 25%. Cette calcination est menée à une température intervenant entre 350 et 600°C et de préférence entre 380 et 550°C et de manière encore plus préférée entre 430 et 550°C.

Mise en oeuvre dans un procédé de déshydrogénation:

Le catalyseur selon l'invention peut être mis en oeuvre dans des procédés de déshydrogénation. Les procédés de déshydrogénation de paraffines légères permettent de valoriser des hydrocarbures aliphatiques à bas point d'ébullition tels que par exemple les butanes et isobutanes, ou les pentanes et isopentanes que l'on peut récupérer après extraction des insaturés des coupes de vapocraquage ou de craquage catalytique. Le procédé de déshydrogénation de paraffines plus longues généralement dans la gamme C8-C16 est un procédé commercial important du fait de la demande actuelle en mono-oléfines pour la préparation de détergents biodégradables ou de produits pharmaceutiques.

Les différents procédés de déshydrogénation de paraffines et de naphtènes se différencient par le choix des conditions opératoires et de la composition de la charge. L'ajustement des conditions opératoires, en fonction de la nature de la charge à traiter, est effectuée de façon à obtenir la meilleure adéquation entre pression, température, rendement, sélectivité, stabilité et activité. Cette adéquation peut être obtenue par des moyens connus de l'homme du métier.

La réaction de déshydrogénation des paraffines est généralement opérée à une pression comprise entre 0,02 et 2 MPa, de préférence comprise entre 0,1 et 1 MPa et à une température comprise entre 400 et 800°C en fonction de la nature de la charge. La température est avantageusement comprise entre 400 et 550°C pour une charge comprenant essentiellement de l'isopentane. La température est avantageusement comprise entre 450 et 550 °C pour une charge comprenant principalement des paraffines comportant de 9 à 22 atomes de carbone par molécule. La charge peut également contenir des hydrocarbures insaturés comportant de 3 à 22 atomes de carbone par molécule.

Le débit massique de charge traitée par unité de masse de catalyseur est généralement compris entre 0,5 et 200 kg/($kg_{cat}$.h). Il peut être avantageux d'utiliser l'hydrogène comme diluant. Le rapport molaire hydrogène/hydrocarbure est généralement compris entre 0 et 20, de préférence entre 0,5 et 10.

La réaction de déshydrogénation des naphtènes est généralement opérée à une pression comprise entre 0,1 et 2 MPa, de préférence comprise entre 0,1 et 1 MPa et à une température comprise entre 200 et 400 °C en fonction de la nature de la charge. Le débit massique de charge traitée par unité de masse de catalyseur est généralement compris entre 0,5 et 100 kg/(kg$_{cat}$.h), de préférence entre 5 et 80 kg/(kg$_{cat}$.h).

Les exemples qui suivent illustrent l'invention.

Exemple 1 (invention) : *Préparation du catalyseur A SnPtSnK/Al2O3*

Un support de billes d'alumine avec un diamètre moyen de 1,2 mm, est préparé par granulation d'une poudre de boehmite, elle-même synthétisée par neutralisation de nitrate d'aluminium par de la soude à pH=10 à 70°C. Après filtration et lavage du gâteau de boehmite, celle-ci est séchée à 100°C pendant 3h. La boehmite est mise en forme par l'injection simultanée sur un plateau granulateur de boehmite et d'eau acidifiée. Les billes ainsi obtenues sont alors séchées à 120°C pendant 5h, puis calcinées à 800°C pendant 4h. La surface BET, déterminée par adsorption d'azote, de ce support est alors de 134 m$^2$/g. Le volume poreux déterminé par porosimétrie mercure est de 0,86 cm$^3$/g.

On prépare un catalyseur A sur ce support en déposant 0,3% poids de platine, 0,4% poids d'étain, 500 ppm poids de chlore et 2 % poids de potassium.

A 100 g de support alumine on ajoute 400 cm$^3$ d'une solution aqueuse contenant du dichlorure d'étain en présence d'acide chlorhydrique. On laisse en contact 3 heures, on filtre, on sèche à 120°C puis on calcine pendant 2 heures à 500°C sous un débit d'air de 100 litres par heure. La quantité de dichlorure d'étain est choisie de façon à obtenir 0,2% poids d'étain sur le produit calciné. On met ensuite le solide en contact avec 500 cm$^3$ d'une solution aqueuse d'acide hexachloroplatinique et d'acide chlorhydrique. On laisse en contact 4 heures puis on essore. On sèche à 90°C puis on met en contact avec 400 cm$^3$ d'une solution aqueuse contenant du tetrachlorure d'étain en présence d'acide chlorhydrique. On laisse en contact 4 heures, on filtre, on sèche à 120°C puis on calcine pendant 2 heures à 500°C sous un débit d'air de 200 litres par heure, avec une vitesse de montée en température de 7°C par minute. La quantité de tetrachlorure d'étain est choisie de façon à obtenir au total 0,4% poids d'étain sur le catalyseur calciné final.

L'indice de bimétallicité et le rapport H$_{ir}$/Pt sont déterminés selon les méthodes décrites dans le descriptif de l'invention.

Le restant du produit est soumis à une déchloration à 540°C sous air saturé avec une solution aqueuse d'ammoniaque concentrée. La durée de ce traitement est ajustée de façon à obtenir 500 ppm poids de chlore sur le catalyseur final. Par la suite, on met en contact avec 500 cm$^3$ d'une solution aqueuse de nitrate de potassium pendant 4 heures de façon à obtenir 2% poids de potassium sur le catalyseur final, on filtre, on sèche à 120°C puis on calcine pendant 2 heures à 500°C sous un débit d'air de 100 litres par heure.

Exemple 2 (invention) : *Préparation du catalyseur B SnPtSnK/Al2O3*

Un catalyseur B est préparé selon le mode opératoire décrit dans l'exemple 1, en choisissant cette fois la quantité de dichlorure d'étain de façon à obtenir 0,27% poids d'étain sur le produit intermédiaire calciné, et la quantité de tétrachlorure d'étain de façon à compléter pour obtenir toujours 0,4% d'étain sur le catalyseur final.

Exemple 3 (comparatif): *Préparation du catalyseur C SnPtSnK/Al2O3*

Un catalyseur C est préparé sur le même support et avec les mêmes teneurs en étain, platine, chlore et potassium qu'en exemple 1.

A 100 g de support alumine on ajoute 400 cm$^3$ d'une solution aqueuse contenant du dichlorure d'étain en présence d'acide chlorhydrique. On laisse en contact 4 heures, on filtre, on sèche à 120°C puis on calcine pendant 2 heures à 500°C sous un débit d'air de 100 litres par heure. La quantité de dichlorure d'étain introduite en une seule étape, est choisie de façon à obtenir 0,4% poids d'étain sur le produit calciné final. On met ensuite le solide en contact avec 400 cm$^3$ d'une solution aqueuse d'acide hexachloroplatinique et d'acide chlorhydrique. On laisse en contact 4 heures puis on essore. On sèche à 120°C puis on calcine pendant 2 heures à 500°C sous un débit d'air de 100 litres par heure, avec une vitesse de montée en température de 7°C par minute.

La détermination de l'indice de bimétallicité et du rapport H$_{ir}$/Pt, ainsi que la déchloration, le dépôt du potassium et la calcination finale se font comme décrit dans l'exemple 1.

Exemple 4 (comparatif): *Préparation d'un catalyseur D SnPtSnK/Al2O3*

Un catalyseur C est préparé selon le mode opératoire décrit dans l'exemple 1, avec pour seule différence que lors des deux imprégnations d'étain, le précurseur utilisé est une solution aqueuse contenant du dichlorure d'étain en présence d'acide chlorhydrique.

Exemple 5 (invention) : *Préparation du catalyseur E SnPt/nSnK/Al2O3*

Un catalyseur E est préparé sur le même support que dans l'exemple 1, en déposant 0,3% poids de platine, 0,4% poids d'étain, 0,2% poids d'indium, 500 ppm poids de chlore et 2 % poids de potassium.

A 100 g de support alumine on ajoute 400 cm$^3$ d'une solution aqueuse contenant du dichlorure d'étain en présence d'acide chlorhydrique. On laisse en contact 4 heures, on filtre, on sèche à 120°C puis on calcine pendant 2 heures à 500°C sous un débit d'air de 100 litres par heure. La quantité de dichlorure d'étain est choisi de façon à obtenir 0,2% poids d'étain sur le produit calciné. On met ensuite le solide en contact avec 400 cm$^3$ d'une solution aqueuse d'acide hexachloroplatinique et d'acide chlorhydrique. On laisse en contact 4 heures puis on essore. On sèche à 90°C puis on met en contact avec 300 cm$^3$ d'une solution aqueuse contenant du nitrate d'indium en présence d'acide chlorhydrique. De nouveau on laisse en contact 4 heures, on essore, on sèche puis on met en contact avec 200 cm$^3$ d'une solution aqueuse contenant du tétrachlorure d'étain en présence d'acide chlorhydrique. On laisse en contact 4 heures, on essore, on sèche à 120°C puis on calcine pendant 2 heures à 500°C sous un débit d'air de 100 litres par heure, avec une vitesse de montée en température de 7°C par minute. La quantité de tétrachlorure d'étain est choisie de façon à obtenir au total 0,4% poids d'étain sur le produit calciné.

La détermination de l'indice de bimétallicité et du rapport $H_{ir}$/Pt, ainsi que la déchloration, le dépôt du potassium et la calcination finale se font comme décrit dans l'exemple 1.

Exemple 6 (invention): *Préparation du catalyseur F PtSnK/(Al2O3-Sn-In)*

Un support de billes d'alumine, contenant 0,2% poids d'étain et 0,2 % poids d'indium, avec un diamètre moyen de 1,2 mm, ayant une surface BET de 130 m2/g, est préparé par granulation de la poudre de boehmite dont la synthèse est décrite dans l'exemple 1 en présence d'eau acidifié, de nitrate d'indium et de Et2Sn(OH)2. Les billes ainsi obtenues sont alors séchées à 120°C pendant 5h, puis calcinées à 800°C pendant 4h. La surface BET, déterminée par adsorption d'azote, de ce support est alors de 130 m$^2$/g. Le volume poreux déterminé par porosimétrie mercure est de 0,88 cm$^3$/g. Un catalyseur F est préparé sur ce support, en déposant 0,3% poids de platine, 0,2% poids supplémentaires d'étain de façon à obtenir 0,4% poids d'étain sur le catalyseur final, 500 ppm poids de chlore et 2 % poids de potassium.

A 100 g du support alumine contenant de l'étain et de l'indium, on ajoute 500 cm$^3$ d'une solution aqueuse d'acide hexachloroplatinique et d'acide chlorhydrique. On laisse en contact 4 heures puis on essore. On sèche à 90°C puis on met en contact avec 500 cm$^3$ d'une solution aqueuse du tetrachlorure d'étain en présence d'acide chlorhydrique. On laisse en contact 4 heures, on essore, on sèche à 120°C puis on calcine pendant 2 heures à 500°C sous un débit d'air de 100 litres par heure, avec une vitesse de montée en température de 7°C par minute. La quantité de tetrachlorure d'étain est choisie de façon à obtenir au total 0,4% poids d'étain sur le produit calciné.

La détermination de l'indice de bimétallicité et du rapport $H_{ir}$/Pt, ainsi que la déchloration, le dépôt du potassium et la calcination finale se font comme décrit dans l'exemple 1.

Exemple 7 (invention) : *Préparation du catalyseur G SnInPtSnK/Al2O3*

Un catalyseur G est préparé sur le même support que dans l'exemple 1, en déposant 0,3% poids de platine, 0,4% poids d'étain, 0,2% poids d'indium et 500 ppm poids de chlore.

A 100 g de support alumine on ajoute 400 cm$^3$ d'une solution aqueuse contenant du dichlorure d'étain en présence d'acide chlorhydrique. On laisse en contact 4 heures, on filtre, on sèche à 120°C puis on calcine pendant 2 heures à 500°C sous un débit d'air de 100 litres par heure. La quantité de dichlorure d'étain est choisi de façon à obtenir 0,2% poids d'étain sur le produit calciné. On met ensuite le solide en contact avec 300 cm$^3$ d'une solution aqueuse contenant du nitrate d'indium en présence d'acide chlorhydrique. De nouveau on laisse en contact 4 heures, on essore et on sèche le solide. On met ensuite le solide en contact avec 400 cm$^3$ d'une solution aqueuse d'acide hexachloroplatinique et d'acide chlorhydrique. On laisse en contact 4 heures puis on essore. On sèche à 90°C puis on met en contact avec 200 cm$^3$ d'une solution aqueuse contenant du tetrachlorure d'étain en présence d'acide chlorhydrique. On laisse en contact 4 heures, on essore, on sèche à 120°C puis on calcine pendant 2 heures à 500°C sous un débit d'air de 100 litres par heure, avec une vitesse de montée en température de 7°C par minute. La quantité de tetrachlorure d'étain est choisie de façon à obtenir au total 0,4% poids d'étain sur le produit calciné.

La détermination de l'indice de bimétallicité et du rapport $H_{ir}$/Pt, ainsi que la déchloration, le dépôt du potassium et la calcination finale, se font comme décrit dans l'exemple 1.

Exemple 8: *Évaluation des performances des catalyseurs A à G en déshydrogénation de n-dodecane*

Les catalyseurs A à G sont soumis à un test de déshydrogénation du n-dodécane réalisé dans un réacteur tubulaire isotherme. 2 g de catalyseur sont réduits à 450 °C durant 2 heures sous un débit de 4 litres par heure d'hydrogène.

Les conditions opératoires sont les suivantes :

- charge : n-dodécane
- température: 430°C pendant 24 heures puis 450°C pendant 24 heures et prélèvement d'échantillons d'effluents pour analyse, puis 470°C avec prélèvement d'échantillons pour analyse après 24 heures, suivies de 120 heures supplémentaires à 470°C, puis 24 heures à 450 °C et prise d'échantillons pour analyse.
- pression: 0,27 MPa
- H2/nC12 (molaire): 5
- débit massique nC12 liquide / masse de catalyseur: 50 h$^{-1}$

Les résultats obtenus après analyse des différents échantillons dans ces conditions sont rapportés dans le tableau 1 ci-dessous. Les valeurs de conversion de nC12 et de rendements sont exprimés en % poids par rapport à la charge.

Tableau 1: Caractéristiques et performances en déshydrogénation de n-dodecane des catalyseurs A à G

| Catalyseur | IBM | H$_{ir}$ / Pt | Température (°C) | Conversion (%) n-C12 | Rendement (%) C12 oléfines | Rendement (%) C12 aromatiques |
|---|---|---|---|---|---|---|
| A (inv.) | 115 | 0,52 | 450 | 10,0 | 9,3 | 0,2 |
|  |  |  | 470 | 14,7 | 13,3 | 0,5 |
|  |  |  | 450 | 9,3 | 8,5 | 0,1 |
| B (inv.) | 111 | 0,55 | 450 | 9,9 | 9,2 | 0,2 |
|  |  |  | 470 | 14,5 | 13,0 | 0,6 |
|  |  |  | 450 | 9,2 | 8,4 | 0,1 |
| C (comp.) | 103 | 0,65 | 450 | 9,9 | 9,1 | 0,3 |
|  |  |  | 470 | 14,1 | 12,3 | 0,7 |
|  |  |  | 450 | 8,5 | 7,7 | 0,1 |
| D (comp.) | 109 | 0,34 | 450 | 7,8 | 7,2 | 0,2 |
|  |  |  | 470 | 10,9 | 9,6 | 0,5 |
|  |  |  | 450 | 6,8 | 6,1 | 0,1 |
| E (inv.) | 130 | 0,50 | 450 | 10,1 | 9,5 | 0,1 |
|  |  |  | 470 | 14,8 | 13,6 | 0,4 |
|  |  |  | 450 | 9,8 | 9,1 | 0,1 |
| F (inv.) | 122 | 0,51 | 450 | 10,0 | 9,3 | 0,2 |
|  |  |  | 470 | 14,8 | 13,4 | 0,6 |
|  |  |  | 450 | 9,6 | 8,8 | 0,1 |
| G (inv.) | 133 | 0,49 | 450 | 10,1 | 9,4 | 0,2 |
|  |  |  | 470 | 14,9 | 13,6 | 0,5 |
|  |  |  | 450 | 9,9 | 9,2 | 0,1 |

Les catalyseurs A, B, E, F et G préparés selon l'invention qui présentent des indices de bimétallicité supérieurs à 108 et des rapports Hir/Pt supérieurs à 0,40, conduisent à des rendements en oléfines supérieurs à ceux des catalyseurs C et D, les catalyseurs C et D préparés selon l'art antérieur. Le gain en rendement des catalyseurs selon l'invention est notamment évident lors du retour à 450°C après passage à 470°, ce qui traduit une plus grande stabilité catalytique des catalyseurs selon l'invention.

**Revendications**

1. Procédé de déshydrogénation d'une charge hydrocarbonée de paraffine à une pression comprise entre 0,02 et 2 MPa, à une température comprise entre 400 et 800°C, à un débit massique de charge traitée par unité de masse de catalyseur compris entre 0,5 et 200 kg/(kgcat.h) et un rapport molaire hydrogène/hydrocarbure compris entre 0 et 20, en présence d'un catalyseur comprenant un métal noble M de platine dans une teneur de 0,01 à 10% pds;

au moins un promoteur X1 constitué par l'étain dans une teneur de 0,005 à 10% pds; un composé alcalin ou alcalino-terreux ; et un support poreux, dans lequel le rapport X1/M est compris entre 0,3 et 8, le rapport $H_{ir}$/M mesuré par chimisorption d'hydrogène de façon irréversible telle que décrite dans la description est supérieur à 0,40 et l'indice IBM de bimétallicité mesuré par titrage hydrogène/oxygène tel que décrit dans la description est supérieur à 108, et dans lequel le procédé de préparation du catalyseur comprend les étapes suivantes:

a) introduction du promoteur X1 dans le support ou sur le support dans une teneur de 30 à 70% pds de la quantité totale de X1,

b) étape optionnelle de séchage du produit obtenu à l'issue de l'étape a,

c) calcination du produit obtenu à l'étape a ou éventuellement à l'étape b à une température de préférence comprise entre 350 et 650°C,

d) dépôt du platine,

e) séchage optionnel sous atmosphère neutre ou contenant de l'oxygène, à une température modérée et n'excédant pas de préférence 150°C,

f) dépôt d'une fraction complémentaire du promoteur X1, dans une teneur de 30 à 70% pds de la quantité totale de X1, sur le produit obtenu à l'étape d ou e, ladite fraction est déposée par mise en contact avec une solution de précurseur de tétrachlorure d'étain,

g) séchage optionnel du produit obtenu à l'étape f,

h) calcination du produit obtenu à l'étape f ou g, cette calcination est de préférence menée en présence d'air et cet air peut également être enrichi en oxygène ou en azote ;

m) deshalogénation en phase gazeuse en présence d'une fraction d'oxygène dépassant les 2% volume, à une température comprise entre 300 et 600°C de façon à obtenir une teneur finale en halogène comprise entre 300 et 1 000 ppm poids ;

n) introduction du composé alcalin ou alcalino-terreux ;

o) séchage optionnel sous une atmosphère neutre ou contenant de l'oxygène, à une température modérée, du produit obtenu à l'étape n ;

p) calcination du produit obtenu à l'étape n ou o, cette calcination est menée en présence d'air et cet air pouvant également être enrichi en oxygène ou en azote.

2. Procédé de déshydrogénation selon la revendication 1 dans lequel le catalyseur comprend en outre au moins un promoteur X2 sélectionné dans le groupe constitué par l'indium le rapport X2/M étant compris entre 0,3 et 8.

3. Procédé de déshydrogénation selon l'une des revendications 1 ou 2 dans lequel le rapport atomique X1/M est compris entre 0,6 et 6.

4. Procédé de déshydrogénation selon la revendication 2 dans lequel le rapport X2/M est compris entre 0,8 et 4.

5. Procédé de déshydrogénation selon la revendication 2 dans lequel le catalyseur comprend un promoteur X1 et un promoteur X2 et dans lequel X1 est l'étain et X2 l'indium.

6. Procédé de déshydrogénation selon l'une des revendications 1 à 5 dans lequel le catalyseur comprend un alcalin sélectionné dans le groupe constitué par : le lithium, le sodium, le potassium, le rubidium et le césium.

7. Procédé de déshydrogénation selon l'une des revendications 1 à 5 dans lequel le catalyseur comprend un alcalin et l'alcalin est le lithium ou le potassium.

8. Procédé de déshydrogénation selon l'une des revendications 1 à 5 dans lequel le catalyseur comprend un alcalino-terreux sélectionné dans le groupe constitué par : le magnésium, le calcium, le strontium ou le baryum.

9. Procédé de déshydrogénation selon l'une des revendications 1 à 5 dans lequel le catalyseur comprend un alcalino-terreux et l'alcalino-terreux est le magnésium ou le calcium.

10. Procédé de déshydrogénation selon l'une des revendications 1 à 9 dans lequel le rapport $H_{ir}$/M du catalyseur est compris entre 0,43 et 0,9.

11. Procédé de déshydrogénation selon l'une des revendications 1 à 9 dans lequel le rapport $H_{ir}$/M du catalyseur est compris entre 0,45 et 0,65.

**12.** Procédé de déshydrogénation selon l'une des revendications 1 à 11 dans lequel l'indice IBM de bimétallicité du catalyseur mesuré par titrage hydrogène/oxygène est compris entre 110 et 160, de préférence compris entre 115 et 145.

**Patentansprüche**

**1.** Dehydrierverfahren einer paraffinischen Kohlenwasserstoffcharge bei einem Druck zwischen 0,02 und 2 MPa, bei einer Temperatur zwischen 400 und 800°C, bei einem Massendurchsatz der behandelten Charge pro Katalysatormasseneinheit zwischen 0,5 und 200 kg/(kgcat.h) und einem Molverhältnis Wasserstoff/Kohlenwasserstoff zwischen 0 und 20, in Gegenwart eines Katalysators, umfassend ein Platinedelmetall M mit einem Gehalt von 0,01 bis 10 Gew.-%; mindestens einen Promotor X1, der aus Zinn besteht, mit einem Gehalt von 0,005 bis 10 Gew.-%; eine Alkali- oder Erdalkali-Verbindung; und einen porösen Träger, wobei das Verhältnis X1/M zwischen 0,3 und 8 liegt, das Verhältnis $H_{ir}/M$, gemessen durch irreversible Chemisorption von Wasserstoff, wie in der Beschreibung beschrieben, größer als 0,40 ist und der Bimetallindex IBM, gemessen durch Wasserstoff/Sauerstofftitration, wie in der Beschreibung beschrieben, größer ist als 108, und wobei das Verfahren zur Herstellung des Katalysators die folgenden Schritte umfasst:

a) Einbringen des Promotors X1 in den Träger oder auf den Träger mit einem Gehalt von 30 bis 70 Gew.-% der Gesamtmenge von X1,
b) optionaler Trocknungsschritt des nach Schritt a erhaltenen Produkts,
c) Calcinierung des in Schritt a oder gegebenenfalls in Schritt b erhaltenen Produkts bei einer Temperatur vorzugsweise zwischen 350 und 650°C,
d) Abscheidung von Platin,
e) optionale Trocknung unter neutraler oder sauerstoffhaltiger Atmosphäre bei einer Temperatur, die gemäßigt ist und vorzugsweise 150°C nicht überschreitet,
f) Abscheidung einer komplementären Fraktion des Promotors X1, mit einem Gehalt von 30 bis 70 Gew.-% der Gesamtmenge von X1, auf dem in Schritt d oder e erhaltenen Produkt, wobei die Fraktion durch Inberührungbringen mit einer Lösung eines Zinntetrachlorid-Vorläufers abgeschieden wird,
g) optionale Trocknung des in Schritt f erhaltenen Produkts,
h) Calcinierung des in Schritt f oder g erhaltenen Produkts, wobei diese Calcinierung vorzugsweise in Gegenwart von Luft durchgeführt wird, und diese Luft auch mit Sauerstoff oder mit Stickstoff angereichert sein kann,
m) Dehalogenierung in der Gasphase in Gegenwart einer Sauerstofffraktion, die 2 Vol.-% überschreitet, bei einer Temperatur zwischen 300 und 600°C, um einen Endgehalt an Halogen zwischen 300 und 1 000 Gew.-ppm zu erhalten,
n) Einbringen der Alkali- oder Erdalkali-Verbindung,
o) optionale Trocknung des in Schritt n erhaltenen Produkts unter einer neutralen oder sauerstoffhaltigen Atmosphäre, bei einer gemäßigten Temperatur,
p) Calcinierung des in Schritt n oder o erhaltenen Produkts, wobei diese Calcinierung in Gegenwart von Luft durchgeführt führt, und diese Luft auch mit Sauerstoff oder mit Stickstoff angereichert sein kann.

**2.** Dehydrierverfahren nach Anspruch 1, wobei der Katalysator außerdem mindestens einen Promotor X2, ausgewählt aus der Gruppe bestehend aus Indium, umfasst, wobei das Verhältnis X2/M zwischen 0,3 und 8 beträgt.

**3.** Dehydrierverfahren nach einem der Ansprüche 1 oder 2, wobei das Atomverhältnis X1/M zwischen 0,6 und 6 beträgt.

**4.** Dehydrierverfahren nach Anspruch 2, wobei das Verhältnis X2/M zwischen 0,8 und 4 beträgt.

**5.** Dehydrierverfahren nach Anspruch 2, wobei der Katalysator einen Promotor X1 und einen Promotor X2 umfasst, und wobei X1 Zinn und X2 Indium ist.

**6.** Dehydrierverfahren nach einem der Ansprüche 1 bis 5, wobei der Katalysator eine Alkali-Verbindung, ausgewählt aus der Gruppe bestehend aus: Lithium, Natrium, Kalium, Rubidium und Cesium, umfasst.

**7.** Dehydrierverfahren nach einem der Ansprüche 1 bis 5, wobei der Katalysator eine Alkali-Verbindung umfasst, und die Alkali-Verbindung Lithium oder Kalium ist.

**8.** Dehydrierverfahren nach einem der Ansprüche 1 bis 5, wobei der Katalysator eine Erdalkali-Verbindung, ausgewählt aus der Gruppe bestehend aus: Magnesium, Calcium, Strontium und Barium, umfasst.

9. Dehydrierverfahren nach einem der Ansprüche 1 bis 5, wobei der Katalysator eine Erdalkali-Verbindung umfasst, und die Erdalkali-Verbindung Magnesium oder Calcium ist.

10. Dehydrierverfahren nach einem der Ansprüche 1 bis 9, wobei das Verhältnis $H_{ir}/M$ des Katalysators zwischen 0,43 und 0,9 beträgt.

11. Dehydrierverfahren nach einem der Ansprüche 1 bis 9, wobei das Verhältnis $H_{ir}/M$ des Katalysators zwischen 0,45 und 0,65 beträgt.

12. Dehydrierverfahren nach einem der Ansprüche 1 bis 11, wobei der Bimetallindex IBM des Katalysators, gemessen durch Wasserstoff/Sauerstofftitration, zwischen 110 und 160, vorzugsweise zwischen 115 und 145, liegt.

**Claims**

1. Process for dehydrogenation of a hydrocarbon feedstock of paraffins at a pressure of between 0.02 and 2 MPa, at a temperature of between 400 and 800°C, a mass rate of the feedstock treated by unit of mass of catalyst between 0.5 and 200 kg($kg_{cat}$.h) and a hydrogen/hydrocarbon molar ratio between 0 and 20, in the presence of a catalyst that comprises a noble metal M of platinum having a content of from 0.01 to 10 % by weight, at least one promoter X1 that consists of tin, having a content of from 0.005 to 10 % by weight, an alkaline or alkaline-earth compound, and a porous substrate, in which the X1/M ratio is between 0.3 and 8, the Hir/M ratio that is measured by hydrogen chemisorption in an irreversible way as described in the specification is more than 0.40, and the bimetallicity index BMI that is measured by hydrogen/oxygen titration as described in the specification is more than 108, and which the process for the preparation of the catalyst comprises the following stages :

   a) introduction of the promoter X1 in the substrate or on the substrate in a content of from 30 to 70% by weight of the total quantity of X1,
   b) optional drying stage of the product that is obtained at the end of stage a),
   c) calcination of the product that is obtained in stage a) or optionally in stage b) at a temperature that is preferably between 350 and 650°C,
   d) deposition of platinum,
   e) optional drying under neutral atmosphere or atmosphere containing oxygen, at a moderate temperature that preferably does not exceed 150°C,
   f) deposition of a complementary fraction of the promoter X1, in a content of from 30 to 70% by weight of the total quantity of X1, on the product that is obtained in stage d) or e), said complementary fraction being deposited by bringing into contact a solution of tin tetrachloride,
   g) optional drying of the product that is obtained in stage f),
   h) calcination of the product that is obtained in stage f) or g), this calcination is preferably conducted in the presence of air, and this air can also be enriched with oxygen or nitrogen, m) deshalogenation in gaseous phase in the presence of an oxygen fraction that exceeds 2% by volume, at temperatures of between 300°C and 600°C so as to obtain a final halogen content of between 300 and 1000 ppm by weight,
   n) introduction of the alkaline or alkaline-earth compound,
   o) optional drying under neutral atmosphere or containing oxygen, at a moderate temperature, of the product obtained in step n),
   p) calcination of the product obtained in step n) or o), said calcination being conducted in the presence of air, and this air can be enriched with oxygen or nitrogen.

2. Dehydrogenation process according to claim 1, in which the catalyst also comprises at least one promoter X2 that is selected from the group that consists of indium, whereby the X2/M ratio is between 0.3 and 8.

3. Dehydrogenation process according to one of claims 1 or 2, in which the XI/M atomic ratio is between 0.6 and 6.

4. Dehydrogenation process according to claim 2, in which the X2/M ratio is between 0.8 and 4.

5. Dehydrogenation process according to claim 2, in which the catalyst comprises a promoter X1 and a promoter X2 and in which X1 is tin and X2 is indium.

6. Dehydrogenation process according to one of claims 1 to 5, in which the catalyst comprises an alkaline that is selected from the group that consists of: lithium, sodium, potassium, rubidium and cesium.

7. Dehydrogenation process according to one of claims 1 to 5, in which the catalyst comprises an alkaline, and the alkaline is lithium or potassium.

8. Dehydrogenation process according to one of claims 1 to 5, in which the catalyst comprises an alkaline-earth that is selected from the group that consists of: magnesium, calcium, strontium or barium.

9. Dehydrogenation process according to one of claims 1 to 5, in which the catalyst comprises an alkaline-earth, and the alkaline-earth is magnesium or calcium.

10. Dehydrogenation process according to one of claims 1 to 9, in which the Hir/M ratio of the catalyst is between 0.43 and 0.9.

11. Dehydrogenation process according to one of claims 1 to 9, in which the Hir/M ratio of the catalyst is between 0.45 and 0.65.

12. Dehydrogenation process according to one of claims 1 to 11, in which the bimetallicity index BMI of the catalyst that is measured by hydrogen/oxygen titration is between 110 and 160, preferably between 115 and 145.

**Figure 1**

## RÉFÉRENCES CITÉES DANS LA DESCRIPTION

**Documents brevets cités dans la description**

- US 4786625 A
- US 20050033101 A1
- US 6600082 B2
- US 6872300 B1
- US 6291394 B1

**Littérature non-brevet citée dans la description**

- **DE G.J. ANTOS ; A.M. AITANI ; J.M. PARERA.** Catalytic Naphta Reforming, Science and Technology. 1995
- **SHARMA et al.** *Applied Catalysis A,* 1998, vol. 168, 251